# EUROPÄISCHE PATENTSCHRIFT

(11) **EP 0 595 771 B1**
(45) Veröffentlichungstag und Bekanntmachung des Hinweises auf die Patenterteilung: **27.11.1996**
(21) Anmeldenummer: 93810743.0
(22) Anmeldetag: 21.10.1993
(51) Int. Cl.: C07D 417/12, C07D 417/14, C10M 135/36, C10N 30/06

(54) **Phosphorfreie Schmiermitteladditive**
Phosphorfree lubricant-additives
Additifs pour lubrifiants exempts de phosphore

(30) Priorität: 30.10.1992 CH 3389/92
(43) Veröffentlichungstag der Anmeldung: 04.05.1994
(73) Patentinhaber: CIBA-GEIGY AG, 4002 Basel (CH)
(72) Erfinder: Camenzind, Hugo, Dr., CH-3014 Bern (CH)

(56) Entgegenhaltungen:
- EP-A- 0 174 262
- EP-A- 0 203 033
- EP-A- 0 259 254
- EP-A- 0 365 127
- DD-A- 154 572
- FR-A- 2 384 282
- INDIAN JOURNAL OF PHARMACEUTICAL SCIENCES Bd. 54, Nr. 3 , Mai 1992 , BOMBAY Seiten 109 - 11 T.P.DABHI ET AL 'Studies on s-triazines:preparation and antimicrobial activity of 2,4-diarylamino-6-(benzthiazol -2-yl-thio)-s-triazines'

## Beschreibung

Die Erfindung betrifft neue heterozyklische Verbindungen und ihre Anwendung als Additive in Schmiermittelzusammensetzungen.

Schmiermitteln werden gewöhnlich eine Reihe von Chemikalien zugesetzt, einerseits um (beispielsweise durch Antioxidantien) die Lebensdauer des Schmiermittels selbst zu erhöhen, andererseits um die zu schmierenden Metallteile vor Abnutzung und Verschleiß zu schützen. Aufgrund ständig steigender Anfordungen an moderne Schmierstoffe und längerer Wechselintervalle sucht man weiter nach neuen, wirksamen Zusatzstoffen. Dabei wird verstärkt Wert auf metall- und phosphorfreie Verbindungen gelegt, da diese günstige Eigenschaften aufweisen, wenn Verbrennungsmotoren mit nachgeschalteten Abgasreinigungskatalysatoren geschmiert werden.

In W.F. Beech, J.Chem.Soc. (C), **1967**, 466 wird die Herstellung von 2,4,6-Tris(benzothiazol-2-ylthio)-1,3,5-triazin beschrieben.

Schmiermitteladditive der Formel 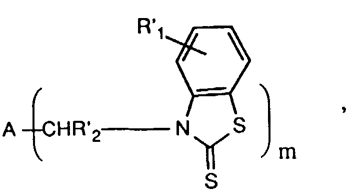 wobei m 1 oder 2 ist und A für -NR'R''', >NR' oder -NR'-R'''-NR'- steht und die Art der Substituenten R'₁, R'₂, R', R'' und R''' hier nicht von Bedeutung ist, sind aus US-A-4,737,302 bekannt.

EP-A-0 365 127 offenbart substituierte Triazine der Formel 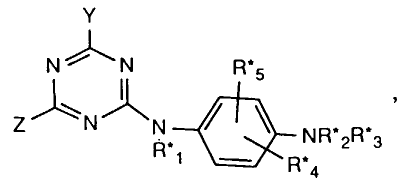 worin Z und Y neben vielen anderen Bedeutungen 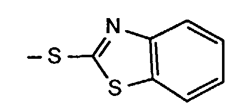 sein können und die Bedeutung der Reste R^{*}₁, R^{*}₂, R^{*}₃, R^{*}₄ und R^{*}₅ hier nicht von Interesse ist. Die Verbindungen sind als Antiozonantien für Gummi wirksam.

DD-A-0 154 572 beschreibt Herbizide der Formel 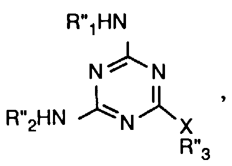 in der R"₁ und R"₂ unabhängig voneinander C₁-C₃-Alkyl und/oder Wasserstoff sind, X für -S- oder -O- steht und R"₃ verschiedentlich substituiertes Phenyl oder Benzothiazolyl-2-reste oder Benzimidazolyl-2-reste sein können.

T.P. Dabhi et al. beschreiben im Indian Journal of Pharmaceutical Sciences **54**, 109-11 (1992) antimicrobiell wirksame Verbindungen der Formel 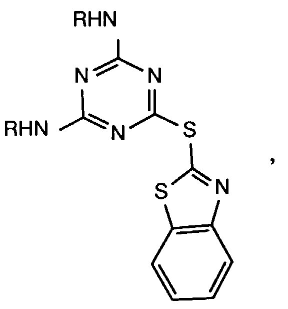 worin R diverse aromatische Reste bedeutet.

In FR-A-2384282 werden polyheterozyklische Stabilisatoren für photographisches Material beschrieben, die der Formel 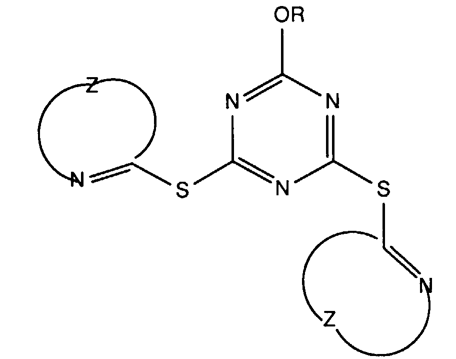 entsprechen, worin Z u.a. eine Gruppe sein kann, die einen Thiazolring vervollständigt.

Es wurde nun gefunden, daß die im folgenden beschriebenen metall- und phosphorfreien Verbindungen sich überraschend gut als Hochdruck- und Verschleißschutzadditive für Schmiermittel eignen sowie als Antioxidantien wirken.

Die Erfindung betrifft daher Verbindungen der Formel I 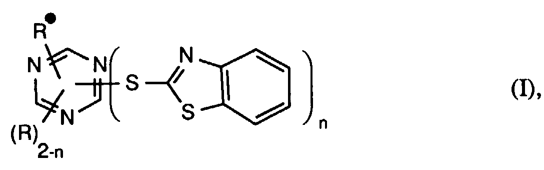 worin
- n: 1 oder 2 ist,
- R und R^{^{●}}: unabhängig voneinander für -OR₁, -SR₂ oder -NR₃R₄ stehen,
- R₁: C₁-C₃₀-Alkyl, C₅-C₇-Cycloalkyl, Phenyl oder Naphthyl, mit Phenyl oder Naphthyl substituiertes C₁-C₁₈-Alkyl oder mit C₁-C₁₂-Alkyl, -Alkoxy oder -Hydroxyalkyl substituiertes Phenyl oder Naphthyl bedeutet,
- R₂: C₁-C₃₀-Alkyl, Phenyl oder Naphthyl, mit Phenyl oder Naphthyl substituiertes C₁-C₁₈-Alkyl oder mit C₁-C₁₂-Alkyl, -Alkoxy oder -Hydroxyalkyl substituiertes Phenyl oder Naphthyl oder ein Rest der Formel (CH₂)ₚ-CO-OR₅ ist,
- p: für 1 oder 2 steht,
R₃ und R₄ unabhängig voneinander die Bedeutungen von R₁ haben, oder -NR₃R₄ für Piperidyl, Pyrrolidyl oder Azepyl steht und
- R₅: Wasserstoff, C₁-C₃₀-Alkyl, C₅-C₇-Cycloalkyl, Phenyl oder Naphthyl, mit Phenyl oder Naphthyl substituiertes C₁-C₁₈-Alkyl oder mit C₁-C₁₂-Alkyl, -Alkoxy oder -Hydroxyalkyl substituiertes Phenyl oder Naphthyl bedeutet.

Bevorzugt sind Verbindungen der Formel I, worin
R₁ C₁-C₁₈-Alkyl bedeutet,
R₂ C₁-C₁₈-Alkyl oder CH₂-CO-OR₅ ist,
R₃ und R₄ unabhängig voneinander C₁-C₁₈-Alkyl oder Benzyl sind, oder -NR₃R₄ für Piperidyl, Pyrrolidyl oder Azepyl steht,
und R₅ C₁-C₁₈-Alkyl bedeutet.

Besonders bevorzugt sind Verbindungen, worin
R und R^{●} unabhängig voneinander für -SR₂ oder -NR₃R₄ stehen,
R₂ C₄-C₁₂-Alkyl ist,
R₃ und R₄ unabhängig voneinander C₁-C₁₂-Alkyl bedeuten und
R₅ C₁-C₁₈-Alkyl ist.

Ganz besonders bevorzugt sind Verbindungen worin n = 2 ist, R₂ für Octyl oder CH₂-CO-O-R₅ steht, und R₁, R₃, R₄, und R₅ n-Octyl, 2-Ethylhexyl oder Isooctyl sind, ebenso Verbindungen, worin n = 1 ist, R und R^{●} für -SR₂ steht und R₂ CH₂-CO-O-iso-Octyl oder CH₂-CO-OR₅ ist,

Sofern n für 1 steht, sind R und R^{●} vorzugsweise gleich.

Alkylsubstituenten in den Verbindungen der Formel I können 1 bis zu 30,insbesondere 1 bis 18, Kohlenstoffatome, enthalten. Beispiele hierfür sind Methyl, Ethyl, Propyl, Butyl, Pentyl, Hexyl, Octyl, Nonyl, Decyl, Undecyl, Dodecyl, Tetradecyl, Hexadecyl, Octadecyl, Eicosyl oder Docosyl sowie entsprechende verzweigte Isomere, insbesondere tert-Butyl, i-Octyl (2-Ethylhexyl) und seine Isomerengemische sowie i-Dodecyl. Alkoxyreste leiten sich in offensichtlicher Weise von diesen Gruppen ab ebenso wie Alkylenreste, die in den Definitionen der in Formel I gezeigten Substituenten enthalten sind.

Ist Alkyl mit Phenyl oder Naphthyl substituiert, so ist bevorzugt Benzyl, Phenethyl, 3-Phenylpropyl, α-Methylbenzyl und α-α-Dimethylbenzyl gemeint. Davon bevorzugt ist Benzyl.

C₅-C₇-Cycloalkyl bedeutet Cyclopentyl, Cyclohexyl oder Cycloheptyl.

Mit C₁-C₁₂-Alkyl, -Alkoxy oder -Hydroxyalkyl substituiertes Phenyl oder Naphthyl kann z.B. Tolyl, Xylyl, Cumyl, Hydroxybenzyl, 3,5-Di-tert-butylhydroxybenzyl oder Methoxybenzyl sein. Bevorzugt sind 1 bis 2 Substituenten am aromatischen Ringsystem vorhanden.

Die Erfindung betrifft ferner Zusammensetzungen enthaltend
A) einen Schmierstoff, eine Hydraulik- oder Metallbearbeitungsflüssigkeit und
B) mindestens eine Verbindung der Formel I.

Komponente A) ist insbesondere ein Schmierstoff.

Die in den erfindungsgemäßen Zusammensetzungen enthaltenen Schmierstoffe, Hydraulik- und Metallbearbeitungsflüssigkeiten können sich mehr oder weniger leicht unter Einfluß von Wärme, Licht bzw. Strahlung, mechanischer Belastung (insbesondere durch Scherkräfte) und chemischen Reagentien (besonders Luftsauerstoff) zersetzen.

Dem Schutz vor solchen Einflüssen dienen die Verbindungen der Formel I, die in den erfindungsgemäßen Zusammensetzungen zweckmäßig zu 0,01 bis 10, beispielsweise zu 0,05 bis 5, vorzugsweise zu 0,05 bis 3, insbesondere jedoch zu 0,1 bis 2 Gew.-% vorliegen sollen. Es kann sich dabei um eine oder mehrere dieser Verbindungen handeln, und die Gewichtsprozentangaben beziehen sich auf die gesamte Menge dieser Verbindungen. Berechnungsgrundlage ist dabei das Gesamtgewicht des Schmierstoffes bzw. der Metallbearbeitungs- oder Hydraulikflüssigkeit ohne die Verbindungen der Formel I.

Die Erfindung betrifft daher auch die Verwendung von Verbindungen der Formel I als Mehrzweckadditive in Schmierstoffen, Hydraulik- und Metallbearbeitungsflüssigkeiten, insbesondere als Antioxidantien, Hochdruck- und Verschleißschutzadditive. Die erfindungsgemäße Verwendung schließt auch den Schutz der zu schmierenden Metallteile vor mechanischer Abnutzung (Verschleißschutz) ein. Eine solche Verwendung bedeutet auch ein Verfahren zur Verbesserung der Gebrauchseigenschaften von Schmierstoffen, Metallbearbeitungs- und Hydraulikflüssigkeiten. Es versteht sich, daß eine erfindungsgemäße Schmierstoffzusammensetzung als Motorenöl verwendet werden kann.

Die in Frage kommenden Schmierstoffe, Metallbearbeitungs- und Hydraulikflüssigkeiten basieren beispielsweise auf mineralischen oder synthetischen Ölen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Öle und Fette, beispielsweise basierend auf einem Mineralöl. Bevorzugt sind Öle.

Eine weitere Gruppe von Schmierstoffen, die zur Anwendung gelangen können, sind pflanzliche oder tierische Öle, Fette, Talge und Wachse oder deren Gemische untereinander oder Gemische mit den erwähnten mineralischen oder synthetischen Ölen. Pflanzliche und tierische Öle, Fette, Talge und Wachse sind beispielsweise Palmkernöl, Palmöl, Olivenöl, Rüböl, Rapsöl, Leinöl, Erdnußöl, Sojabohnenöl, Baumwollöl, Sonnenblumenöl, Kürbiskernöl, Kokosnußöl, Maisöl, Rizinusöl, Baumnußöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-α-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-α-Olefine, Schmierstoffe auf Esterbasis, Phosphate, Glykole, Polyglykole und Polyalkylenglykole, sowie deren Mischungen mit Wasser.

Metallbearbeitungsflüssigkeiten und Hydraulikflüssigkeiten können auf Basis der gleichen Substanzen hergestellt werden wie vorstehend für die Schmiermittel beschrieben. Häufig handelt es sich dabei auch um Emulsionen solcher Substanzen in Wasser oder anderen Flüssigkeiten.

Erfindungsgemäße Schmierstoffzusammensetzungen finden z.B. Verwendung in Verbrennungsmotoren, z.B. in Kraftfahrzeugen.

Die Verbindungen der Formeln I sind gut in Schmierstoffen, Metallbearbeitungs- und Hydraulikflüssigkeiten löslich und sind deshalb als Zusätze zu Schmierstoffen, Metallbearbeitungs- und Hydraulikflüssigkeiten besonders geeignet, und es ist besonders auf ihre überraschend gute antioxidative und verschleißhemmende Wirkung hinzuweisen.

Daher betrifft die vorliegende Erfindung ebenfalls ein Verfahren zur Verbesserung der Gebrauchseigenschaften von Schmierstoffen, Metallbearbeitungs- und Hydraulikflüssigkeiten, dadurch gekennzeichnet, daß diesen Verbindungen der Formel I zugesetzt werden.

Die erfindungsgemäßen Schmierstoffe, Metallbearbeitungs- und Hydraulikflüssigkeiten können zusätzlich andere Additive enthalten, die zugegeben werden, um ihre Grundeigenschaften noch weiter zu verbessern; dazu gehören: Korrosionsschutzmittel, Rostinhibitoren, Metall-Desaktivatoren, Viskositätsindex-Verbesserer, Dispergiermittel, weitere Antioxidantien, Stockpunkterniedriger, Hochdruck- und Verschleißschutzzusätze, Detergentien, weitere Hochdruck-Zusätze und Antiverschleiß-Additive.
Es folgen Beispiele solcher weiteren Zusatzstoffe:

### Beispiele für phenolische Antioxidantien

1. Alkylierte Monophenole, z.B. 2,6-Di-tert.butyl-4-methylphenol, 2-Tert.butyl-4,6-di-methylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Di-methyl-6-(1'-methyl-tridec-1'-yl)-phenol und Mischungen davon.
2. Alkylthiomethylphenole, z.B. 2,4-Di-octylthiomethyl-6-tert.-butylphenol, 2,4-Di-octylthiomethyl-6-methylphenol, 2,4-Di-octylthiomethyl-6-ethylphenol, 2,6-Di-dodecylthiomethyl-4-nonylphenol.
3. Hydrochinone und alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol, 2,6-Di-tert.-butyl-hydrochinon, 2,5-Di-tert.-butyl-4-hydroxyanisol, 3,5-Di-tert.-butyl-4-hydroxyanisol, 3,5-Di-tert.-butyl-4-hydroxyphenyl-stearat, Bis-(3,5-di-tert.-butyl-4-hydroxyphenyl)adipat.
4. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol), 4,4'-Thio-bis-(3,6-di-sec.-amylphenol), 4,4'-Bis(2,6-dimethyl-4-hydroxyphenyl)-disulfid.
5. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylenbis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(a-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis-[3,3-bis-(3'-tert.-butyl-4'-hydroxyphenyl)-butyrat], Bis-(3-tert.butyl-4-hydroxy-5-methyl-phenyl)-dicyclopentadien, Bis-[2-(3'-tert.butyl-2'-hydroxy-5'-methyl-benzyl)-6-tert.butyl-4-methyl-phenyl]-terephthalat, 1,1-Bis-(3,5-dimethyl-2-hydroxyphenyl)-butan, 2,2-Bis-(3,5-di-tert.-butyl-4-hydroxyphenyl)-propan, 2,2-Bis-(5-tert.-butyl-4-hydroxy-2-methylphenyl)-4-n-dodecylmercapto-butan, 1,1,5,5-Tetra-(5-tert.-butyl-4-hydroxy-2-methylphenyl)-pentan.
6. O-, N- und S-Benzylverbindungen, z.B. 3,5,3',5'-Tetra-tert.-butyl-4,4'-dihydroxydibenzylether, Octadecyl-4-hydroxy-3,5-dimethylbenzyl-mercaptoacetat, Tris-(3,5-di-tert.-butyl-4-hydroxybenzyl)-amin, Bis-(4-tert.-butyl-3-hydroxy-2,6-dimethylbenzyl)-dithioterephthalat, Bis-(3,5-di-tert.-butyl-4-hydroxybenzyl)-sulfid, Isooctyl-3,5-di-tert.-butyl-4-hydroxybenzyl-mercaptoacetat.
7. Hydroxybenzylierte Malonate, z.B. Dioctadecyl-2,2-bis-(3,5-di-tert.-butyl-2-hydroxybenzyl)-malonat, Di-octadecyl-2-(3-tert.-butyl-4-hydroxy-5-methylbenzyl)-malonat, Didodecylmercaptoethyl-2,2-bis-(3,5-di-tert.-butyl-4-hydroxybenzyl)-malonat, Di-[4-(1,1,3,3-tetramethylbutyl)-phenyl]-2,2-bis-(3,5-di-tert.-butyl-4-hydroxybenzyl)-malonat.
8. Hydroxybenzyl-Aromaten, z.B. 1,3,5-Tris-(3,5-di-tert.-butyl-4-hydroxybenzyl)-2,4,6-trimethylbenzol, 1,4-Bis-(3,5-di-tert.-butyl-4-hydroxybenzyl)-2,3,5,6-tetramethylbenzol, 2,4,6-Tris-(3,5-di-tert.-butyl-4-hydroxybenzyl)-phenol.
9. Triazinverbindungen, z.B. 2,4-Bis-octylmercapto-6-(3,5-di-tert.-butyl-4-hydroxyanilino)-1,3,5-triazin, 2-Octylmercapto-4,6-bis-(3,5-di-tert.-butyl-4-hydroxyanilino)-1,3,5-triazin, 2-Octylmercapto-4,6-bis-(3,5-di-tert.-butyl-4-hydroxyphenoxy) -1,3,5-triazin, 2,4,6-Tris-(3,5-di-tert.-butyl-4-hydroxyphenoxy)-1,2,3-triazi n, 1,3,5-Tris-(3,5-di-tert.-butyl-4-hydroxybenzyl)-isocyanurat, 1,3,5-Tris-(4-tert.-butyl-3-hydroxy-2,6-dimethylbenzyl)-isocyanurat, 2,4,6-Tris-(3,5-di-tert.-butyl-4-hydroxyphenylethyl)-1,3,5-triazin, 1,3,5-Tris-(3,5-di-tert.butyl-4-hydroxyphenylpropionyl)-hexahydro-1,3,5-triazin, 1,3,5-Tris-(3,5-dicyclohexyl-4-hydroxybenzyl)-isocyanurat.
10. Benzylphosphonate, z.B. Dimethyl-2,5-di-tert.-butyl-4-hydroxybenzylphosphonat, Diethyl-3,5-di-tert.-butyl-4-hydroxybenzylphosphonat, Dioctadecyl-3,5-di-tert.-butyl-4-hydroxybenzylphosphonat, Dioctadecyl-5-tert.-butyl-4-hydroxy-3-methylbenzylphosphonat, Ca-Salz des 3,5-Di-tert.-butyl-4-hydroxybenzyl-phosphonsäure-monoethylest ers.
11. Acylaminophenole, z.B. 4-Hydroxy-laurinsäureanilid, 4-Hydroxystearinsäureanilid, N-(3,5-di-tert.butyl-4-hydroxyphenyl)-carbaminsäureoctylester.
12. Ester der β-(3,5-Di-tert.butyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
13. Ester der β-(5-tert.Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
15. Ester der 3,5-Di-tert.-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
16. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.-butyl-4-hydroxyphenylpropionyl)-trimethylendiamin, N,N'-Bis-(3,5-di-tert.butyl-4-hydroxyphenylpropionyl)-hydrazin.

### Beispiele für aminische Antioxidantien:

N,N'-Di-isopropyl-p-phenylendiamin, N,N'-Di-sec-butyl-p-phenylendiamin, N,N'-Bis-(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'- phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methylphenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Isopropyl/Isohexyl-diphenylaminen, Gemische aus mono- und dialkylierten tert.-Butyldiphenylaminen, 2,3-Dihydro-3,3-dimethyl-4H-1,4-benzothiazin, Phenothiazin, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

### Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,12-dihydroxy-3,7,10, 14-tetrathiahexadecan.

### Beispiele für Metall-Desaktivatoren. z.B. für Kupfer, sind:

a) Benzotriazole und deren Derivate, z.B. 4- oder 5-Alkylbenzotriazole (z.B. Tolutriazol) und deren Derivate, 4,5,6,7-Tetrahydrobenzotriazol, 5,5'-Methylenbis-benzotriazol; Mannich-Basen von Benzotriazol oder Tolutriazol wie 1-[Di(2-ethylhexyl)aminomethyl)-tolutriazol und 1-[Di(2-ethylhexyl)aminomethyl)-benzotriazol; Alkoxyalkylbenzotriazole wie 1-(Nonyloxymethyl)-benzotriazol, 1-(1-Butoxyethyl)-benzotriazol und 1-(1-Cyclohexyloxybutyl)-tolutriazol.
b) 1,2,4-Triazole und deren Derivate, z.B. 3-Alkyl (oder Aryl)- 1,2,4-Triazole, Mannich-Basen von 1,2,4-Triazolen wie 1-[Di(2-ethylhexyl)aminomethyl-1,2,4-triazol; Alkoxyalkyl-1,2,4-triazole wie 1-(1-Butoxyethyl-1,2,4-triazol; acylierte 3-Amino-1,2,4-triazole.
c) Imidazolderivate, z.B. 4,4'-Methylenbis(2-undecyl-5-methylimidazol, Bis[(N-methyl)imidazol-2-yl]carbinol-octylether.
d) Schwefelhaltige heterocyclische Verbindungen, z.B. 2-Mercaptobenzothiazol, 2,5-Dimercapto-1,3,4-thiadiazol und deren Derivate; 3,5-Bis[di(2-ethylhexyl)amino-methyl]-1,3,4-thiadiazolin-2-on.
e) Aminoverbindungen, z.B. Salicyliden-propylendiamin, Salicylaminoguanidin und deren Salze.

### Beispiele für Rost-Inhibitoren sind:

a) Organische Säuren, ihre Ester, Metallsalze, Aminsalze und Anhydride, z.B. Alkyl- und Alkenyl-bernsteinsäuren und deren Partialester mit Alkoholen, Diolen oder Hydroxycarbonsäuren, Partialamide von Alkyl- und Alkenylbernsteinsäuren, 4-Nonylphenoxyessigsäure, Alkoxy- und Alkoxyethoxycarbonsäuren wie Dodecyloxyessigsäure, Dodecyloxy(ethoxy)-essigsäure und deren Aminsalze, ferner N-Oleoyl-sarcosin, Sorbitan-mono-oleat, Blei-naphthenat, Alkenylbernsteinsäureanhydride, z.B. Dodecenylbernsteinsäure-anhydrid, 2-(2-Carboxymethyl)-1-dodecyl-3-methylglycerin und dessen Salze, Aminsalze.
b) Stickstoffhaltige Verbindungen, z.B.:
   I. Primäre, sekundäre oder tertiäre aliphatische oder cycloaliphatische Amine und Amin-Salze von organischen und anorganischen Säuren, z.B. öllösliche Alkylammoniumcarboxylate, ferner 1-[N,N-bis-(2-hydroxyethyl)amino]-3-(4-nonylphenoxy)propan-2-ol.
   II. Heterocyclische Verbindungen, z.B.:
      Substituierte Imidazoline und Oxazoline, 2-Heptadecenyl-1-(2-hydroxyethyl)-imidazolin.
   c) Phosphorhaltige Verbindungen, z.B.:
      Aminsalze von Phosphorsäurepartialestern oder Phosphonsäurepartialestern, Zinkdialkyldithiophosphate.
   d) Schwefelhaltige Verbindungen, z.B.:
      Barium-dinonylnaphthalin-sulfonate, Calciumpetroleum-sulfonate, Alkylthio-substituierte aliphatische Carbonsäuren, Ester von aliphatischen 2-Sulfocarbonsäuren und deren Salze.
   e) Glycerinderivate, z.B.:
      Glycerin-monooleat, 1-(Alkylphenoxy)-3-(2-hydroxyethyl)glycerine, 1-(Alkylphenoxy)-3-(2,3-dihydroxypropyl)glycerine, 2-Carboxyalkyl-1,3-dialkylglycerine.

### Beispiele für Viskositätsindex-Verbesserer sind:

Polyacrylate, Polymethacrylate, Vinylpyrrolidon/Methacrylat-Copolymere, -Copolymere, Polyvinylpyrrolidone, Polybutene, Olefin-Copolymere, Styrol/Acrylat-Copolymere, Polyether.

### Beispiele für Stockpunkterniedriger sind:

Polymethacrylat, alkylierte Naphthalinderivate.

### Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

### Beispiele für Verschleißschutz-Additive sind:

Schwefel und/oder Phosphor und/oder Halogen enthaltende Verbindungen, wie geschwefelte Olefine und pflanzliche Öle, Zinkdialkyldithiophosphate, Tritolylphosphat, Tricresylphosphat, chlorierte Paraffine, Alkyl- und Aryldi- und tri-sulfide, Aminsalze von Mono- und Dialkylphosphaten, Aminsalze der Methylphosphonsäure, Diethanolaminomethyltolyltriazol, Di(2-ethylhexyl)aminomethyltolyltriazol, Derivate des 2,5-Dimercapto-1,3,4-thiadiazols, 3-[(Bis-isopropyloxy-phosphinothioyl)thio]-propionsäureethylester, Triphenylthiophosphat (Triphenylphosphorothioat), Tris(alkylphenyl)phosphorothioate und deren Gemische, (z.B. Tris(isononylphenyl)phosphorothioat), Diphenyl-monononylphenyl-phosphorothioat, Isobutylphenyl-diphenyl-phosphorothioat, Dodecylaminsalz des 3-Hydroxy-1,3-thiaphosphetan-3-oxids, Trithiophosphorsäure-5,5,5-tris[isooctylacetat (2)], Derivate von 2-Mercaptobenzothiazol wie 1-[N,N-Bis(2-ethylhexyl)aminomethyl-2-mercapto-1H-1,3-benzothiazol, Ethoxycarbonyl-5-octyl-dithiocarbamat.

Die Verbindungen der Formel I sind neu.

Die Umsetzung von Cyanurchlorid mit verschiedenen protischen Reaktanden, die >N-H, -OH oder -SH Gruppen enthalten, ist bekannt (W.F. Beech, J. Chem. Soc. C, 1967 466-72). Da diese Substituenten gewöhnlich eine desaktivierende Wirkung auf die weiteren reaktiven Positionen des Cyanurchlorids ausüben, ist es möglich, die Chloratome schrittweise zu ersetzen. Verschiedene Lösungsmittel wie Aceton, Benzol oder auch Wasser, können zweckmäßig sein, gegebenenfalls auch Zweiphasengemische. Bewährt haben sich Aceton bzw. Aceton-Wasser-Gemische.

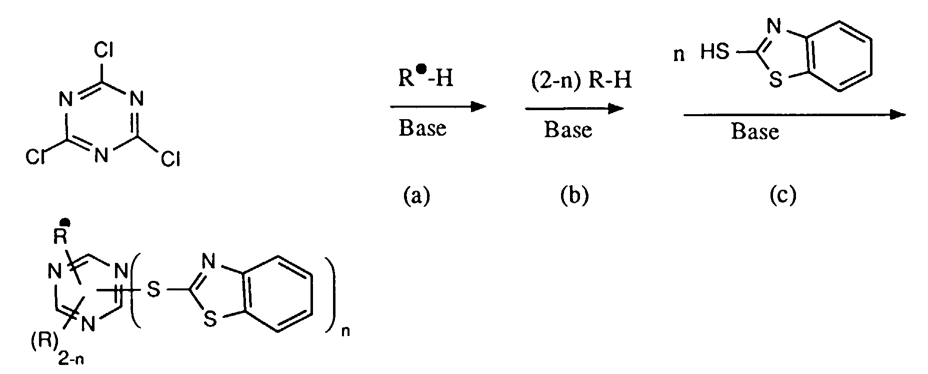

Die Verbindungen der Formel I, bei denen R und R^{●} gleich sind [(a) und (b) bilden im obigen Schema zusammen eine Stufe)] oder n 2 bedeutet [(b) fällt weg], werden daher ausgehend vom Cyanurchlorid in zweistufiger Synthese hergestellt. Das Cyanurchlorid wird dabei mit 1 oder 2 Moläquivalent Amin, Mercaptan oder Alkohol in einem geeigneten Lösungmittel, z.B. Aceton, gelöst und bei 0-5°C mit 1 oder 2 Moläquivalent einer Base (z.B. tertiäres Amin, KOH oder NaOH) behandelt. Das Mono- oder Dichlorzwischenprodukt kann dann in Lösung mit 2 oder 1 Moläquivalent 2-Mercaptobenzothiazol und entsprechend 1 oder 2 Moläquivalent Base bei Raumtemperatur bis 60°C weiter umgesetzt werden, wobei die Base zweckmäßig in geringem Überschuß zum Einsatz kommt. Sind R und R^{●} verschieden, so wird eine Stufe mehr benötigt [Schritte (a), (b) und (c) im Schema]. Sofern R bzw. R^{●} für NR₃R₄ steht, wird zweckmäßig zuerst mit 1 Aequivalent Amin umgesetzt, weil dies stärker desaktiviert als OH- oder SH-Verbindungen.

Die als Ausgangsstoffe verwendeten -SH, -OH und >NH-Verbindungen sind an sich bekannt oder nach an sich bekannten Verfahren herstellbar bzw. kommerziell erhältlich.

Die folgenden Beispiele erläutern die Erfindung weiter. Alle Angaben in Prozenten oder Teilen beziehen sich auf das Gewicht, sofern nicht anders angegeben. i-Octyl bedeutet die Alkylreste eines als 2-Mercaptoisooctylacetat erhältlichen Isomerengemisches.

### Beispiel 1

2,4-Di-(2-mercaptobenzothiazol-S-yl)-6-(N,N-di-2-ethylhexylamino)-1,3,5-triazin

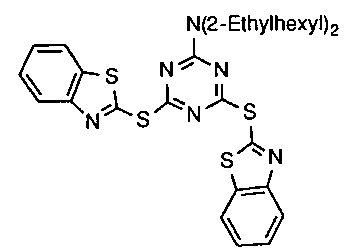

Zu einer Lösung von 147.5 g Cyanurchlorid in 21 Aceton werden innerhalb von 10 min bei 0-5°C 245 ml Di(2-ethylhexyl)amin zugetropft. Innerhalb weiterer 10 min werden 400 ml 2 N Natronlauge bei 0-5°C zugetropft. Nach weiteren 20 min Rühren bei 0-5°C wird auf 2 l Wasser gegossen, mit 1 l Hexan extrahiert und das Extrakt eingedampft. Man erhält 304 g 2,4-Dichlor-6-(N,N-di-2-ethylhexylamino)-1,3,5-triazin als hellgelbes Öl.

Zu einer Lösung von 4.1 g der oben erhaltenen Verbindung in 30 ml Ethanol/Wasser 3:1 werden 4.2 g 2-Mercaptobenzothiazol und anschließend eine Lösung von 1 g NaOH in 10 ml Wasser zugegeben. Das Reaktionsgemisch wird 15 h bei Rückfluß gerührt. Dann wird im Scheidetrichter mit Wasser verdünnt und mit Hexan extrahiert. Die Hexanphase wird mit etwas Natronlauge 2N, Sole und Wasser gewaschen, eingedampft und das Rohprodukt über eine kurze Säule mit 120 g Silicagel (Gradient Hexan - Toluol - 2% Ethanol) gereinigt. Nach Entfernung der Lösungsmittel erhält man

4.1 g klares, hellgelbes, viskoses Öl (63% d.Th.).

| Mikroanalyse: | C | H | N | S |
|---|---|---|---|---|
| berechnet | 60.89% | 6.50% | 12.91% | 19.74% |
| gefunden | 61.65% | 6.92% | 12.34% | 18.76% |

### Beispiel 2: 2,4-Di-(2-mercaptobenzothiazol-S-yl)-6-(n-octylthio)-1,3,5-triazin

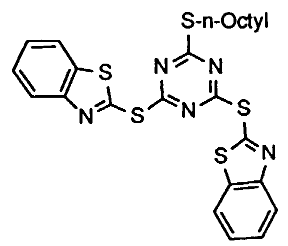

Zu einer Lösung von 188.2 g Cyanurchlorid in 1.5 l Aceton werden 151 g n-Octanthiol zugegeben, gefolgt von 510 ml Natronlauge 2 N bei 0-5°C innerhalb von 30 min. Die Emulsion wird noch 1 h bei 0-5°C und eine weitere Stunde bei 5-15°C gerührt, auf Wasser gegossen und mit 500 ml Toluol extrahiert. Nach Waschen des Extraktes mit Sole und Wasser wird das Lösungsmittel eingedampft. Es verbleiben 265 g 2,4-Dichlor-6-(n-octanthiol-S-yl)-1,3,5-triazin als klares, hellgelbes Öl (90%d.Th.).

124 g davon werden in 1 l Aceton gelöst. Nacheinander werden 155 g 2-Mercaptobenzothiazol und 480 ml 2 N Natronlauge bei 22-32°C innerhalb von 30 min zugegeben. Nach einer Stunde Nachrühren bei Raumtemperatur wird auf 1 l Sole gegossen und mit 500 ml Toluol extrahiert. Nach Waschen der organischen Phase mit Sole und Wasser wird das Lösungsmittel entfernt. Das Rohprodukt (210 g) wird in 200 ml Toluol gelöst und unter Rühren langsam mit 3 mal 300 ml Hexan gemischt. Innerhalb 30 min kristallisiert das Produkt. Nach weiteren 30 Minuten wird abgenutscht und mit etwas Hexan nachgewaschen: Ausbeute 185 g leicht hellgelbe Kristalle (83% d.Th.), Smp. 75-77°C.

| Mikroanalyse: | C | H | N | S |
|---|---|---|---|---|
| berechnet | 54.02% | 4.53% | 12.60% | 28.84% |
| gefunden | 54.85% | 4.53% | 12.21% | 28.09% |

### Beispiel 3

2,4-Di-(2-mercaptobenzothiazol-S-yl)-6(2-mercapto-iso-octylacetat-S-yl)-1,3,5-triazin

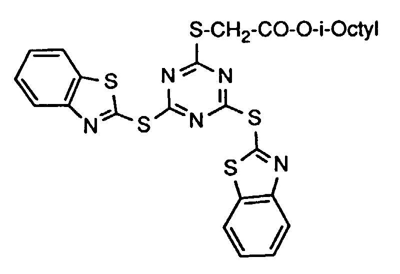

Zu einer Lösung von 92.2 g Cyanurchlorid in 1 l Aceton werden 102.1 g 2-Mercapto-iso-octylacetat (Isomerengemisch) zugegeben, gefolgt von 61.8 g Collidin bei -20°C innerhalb von 20 min. Innerhalb von 30 min wird auf 0-5°C erwärmt, auf 1.5 l Eiswasser gegossen und mit 1 l Hexan extrahiert. Nach Eindampfen des Lösungsmittels wird das Rohprodukt über eine kurze Säule mit 400 g Silicagel (Gradient Hexan -Toluol) gereinigt. Nach Entfernung des Lösungsmittels verbleiben 142 g 2,4-Dichlor-6-(2-mercapto-iso-octylacetat-S-yl)-1,3,5-triazin als leicht orangefarbenes Öl (81% d.Th.).

Zu einer Lösung von 17.6 g dieses Öls in 400 ml Aceton werden 18.4 g 2-Mercaptobenzothiazol gegeben und anschließend bei Raumtemperatur bis 30°C innerhalb von 15 min 16.7 ml Triethylamin zugetropft. Nach einer Stunde Rühren bei Raumtemperatur wird auf 500 ml Eiswasser gegossen und mit 500 ml Hexan und 500 ml Toluol extrahiert. Die organische Phase wird mit etwas 2N Natronlauge, Sole und Wasser gewaschen und eingedampft, das Rohprodukt über eine kurze Säule mit 200 g Silicagel (Gradient Toluol - 1% Ethylacetat) gereinigt. Nach Einengen erhält man 19.7 g (64% d.Th.) hellorangefarbenes, Kristallisat, Smp. 75-78°C.

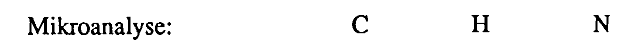

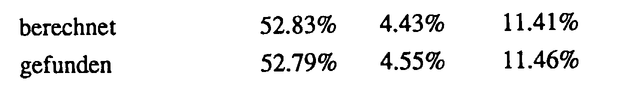

### Beispiel 4

2-(2-Mercaptobenzothiazol-S-yl)-4,6-di-(2-mercapto-iso-octylacetat-S-yl)-1,3,5-triazin

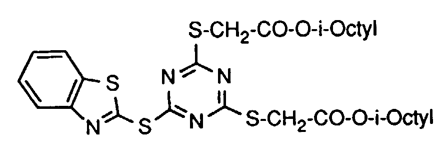

Zu einer Lösung von 92.2 g Cyanurchlorid in 1 l Aceton werden 102.1 g 2-Mercapto-iso-octylacetat (Isomerengemisch) zugegeben, gefolgt von 61.8 g Collidin bei -20°C innerhalb von 20 min. Innerhalb von 30 min wird auf 0-5°C erwärmt. Wieder werden 102.1 g 2-Mercapto-iso-octylacetat (Isomerengemisch) zugegeben, gefolgt von 61.8 g Collidin bei -20°C innerhalb von 20 min. Die orangefarbene Suspension wird 10 h bei Raumtemperatur weitergerührt, auf 1 l Wasser gegossen und mit 500 ml Toluol extrahiert. Nach Eindampfen des Lösungsmittels wird das Rohprodukt über eine kurze Säule mit 400 g Silicagel (Gradient Hexan -Toluol) gereinigt. Nach Etnfernung der Lösungsmittel verbleiben 245 g
2-Chlor-4,6-di-(2-mercapto-iso-octylacetat-S-yl)-1,3,5-triazin als gelbes, mittelviskoses Öl (94% d.Th.).

Zu einer Lösung von 26 g dieser Verbindung in 300 ml Toluol werden 9.2 g Mercaptobenzothiazol und anschließend 9 ml Triethylamin zugegeben. Die klare braune Lösung wird auf 60°C erwärmt und 30 min bei dieser Temperatur gerührt. Die Suspension wird nacheinander mit Wasser, etwas 2 N Natronlauge, Sole und Wasser gewaschen und eingedampft. Das Rohprodukt wird über eine kurze Säule mit 400 g Silicagel gereinigt (Gradient Hexan-Toluol-2% Ethylacetat). Nach Entfernung des Laufmittels erhält man:
21.5 g klares, hellgelbes, viskoses Öl (66% d.Th.).

| Mikroanalyse: | C | H | N | S |
|---|---|---|---|---|
| berechnet | 60.89% | 6.50% | 12.91% | 19.74% |
| gefunden | 61.65% | 6.92% | 12.34% | 18.76% |

### Beispiel 5: Test auf Verschleißschutz

Zur Prüfung auf Eignung als Verschleißschutzadditiv wird die ASTM-Standardmethode D-2783-81 unter Verwendung des Shell-Vierkugelapparates herangezogen. Als Basisöl wird STOCK 305 der Fa. Mobil verwendet, dem die in der Tabelle angegebene Menge an Verbindung gemäß dem jeweils genannten Beispiel zugegeben wird. Ermittelt werden:
a) die Schweißlast WL (Weld Load) als die Last (in kg), bei der die Kugeln innerhalb von 10 s zusammenschweißen, sowie
b) der mittlere Verschleiß-Narben-Durchmesser WSD (Wear-Scar-Diameter) bei einer Last von 20 kg nach einer Stunde Betrieb bei 60°C (in mm).

Die erhaltenen Resultate sind in Tabelle I aufgeführt.

**Tabelle I:**

| Verbindung aus Beispiel | Zusatzmenge [%] | WL [kg] | WSD [mm] |
|---|---|---|---|
| ohne Zusatz | | 130 | 0.82 |
| 1 | 1.0 | 160 | 0.50 |
| 2 | 1.0 | 160 | 0.56 |
| 3 | 1.0 | 160 | 0.56 |
| 4 | 1.0 | 160 | 0.55 |

### Beispiel 6: Test auf Stabilisierung gegen oxidativen Abbau

### (TFOUT: Thin Film Oxygen Uptake Test)

Dieser Test ist eine Modifikation des Rotary Bomb Oxidation Test for Mineral Oils (ASTM D 2272) und ist vom National Bureau of Standards (NBS), Washington D.C., anerkannt. Er wird näher beschrieben in "C.S. Ku, S. M. Hsu, Lubrication Engineering 40, 75-83 (1984)". Als Testöl wird kommerziell erhältliches Motorenöl vom Typ 15 W 40 verwendet, das ca. die Hälfte des üblichen Gehalts an Zinkdialkyldithiophosphat enthält (0.75% ZnDTP, 550 ppm P, 1160 ppm Zn).

Das zu testende Additiv wird zu 0.5 Gew.% im Testöl gelöst und in Gegenwart von 2% Wasser, 4% einer oxidierten und nitrierten Benzinfraktion und 4% eines flüssigen Metallnaphthenates als Katalysatoren (erhältlich unter Nr. 1817 vom NBS) im Ölbad unter einem Sauerstoffanfangsdruck von 610 kPa auf 160°C erhitzt. Durch Rotieren bei 100 Upm in einem axialen Winkel von 30° wird ein Ölfilm an der Innenwand des Glasbehälters gebildet. Nach einer sog. Induktionszeit beginnt das Öl zu oxidieren, was sich an einem rapiden Abfall des Sauerstoffdrucks zeigt. Das Testöl selbst wird der gleichen Prozedur unterzogen. Als Testergebnis wird die Induktionszeit festgehalten. Je länger diese ist, desto stabiler ist die Ölmischung gegen Oxidation.

Die Ergebnisse sind der folgenden Tabelle II zu entnehmen:

**Tabelle II:**

| Verbindung aus Beispiel | Zusatzmenge [%] | Induktionsperiode [min] |
|---|---|---|
| ohne Zusatz | - | 83 |
| 1 | 0.5 | 117 |
| 2 | 0.5 | 152 |
| 3 | 0.5 | 151 |
| 4 | 0.5 | 133 |

## Patentansprüche

1. Verbindungen der Formel I 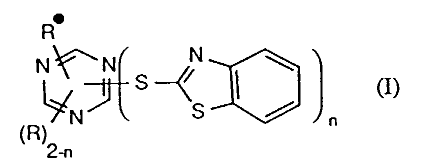 worin
n 1 oder 2 ist,
R und R^{●} unabhängig voneinander für -OR₁, -SR₂ oder -NR₃R₄ stehen,
R₁ C₁-C₃₀-Alkyl, C₅-C₇-Cycloalkyl, Phenyl oder Naphthyl, mit Phenyl oder Naphthyl substituiertes C₁-C₁₈-Alkyl oder mit C₁-C₁₂-Alkyl, -Alkoxy oder -Hydroxyalkyl substituiertes Phenyl oder Naphthyl bedeutet,
R₂ C₁-C₃₀-Alkyl, Phenyl oder Naphthyl, mit Phenyl oder Naphthyl substituiertes C₁-C₁₈-Alkyl oder mit C₁-C₁₂-Alkyl, -Alkoxy oder -Hydroxyalkyl substituiertes Phenyl oder Naphthyl oder ein Rest der Formel (CH₂)ₚ-CO-OR₅ ist,
p für 1 oder 2 steht,
R₃ und R₄ unabhängig voneinander die Bedeutungen von R₁ haben, oder -NR₃R₄ für Piperidyl, Pyrrolidyl oder Azepyl steht und
R₅ Wasserstoff, C₁-C₃₀-Alkyl, C₅-C₇-Cycloalkyl, Phenyl oder Naphthyl, mit Phenyl oder Naphthyl substituiertes C₁-C₁₈-Alkyl oder mit C₁-C₁₂-Alkyl, -Alkoxy oder -Hydroxyalkyl substituiertes Phenyl oder Naphthyl bedeutet.

2. Verbindungen nach Anspruch 1, worin
R₁ C₁-C₁₈-Alkyl bedeutet,
R₂ C₁-C₁₈-Alkyl oder CH₂-CO-OR₅ ist,
R₃ und R₄ unabhängig voneinander, C₁-C₁₈-Alkyl oder Benzyl sind, oder -NR₃R₄ für Piperidyl, Pyrrolidyl oder Azepyl steht,
und R₅ C₁-C₁₈-Alkyl bedeutet.

3. Verbindungen nach Anspruch 2, worin R und R^{●} unabhängig voneinander für -SR₂ oder -NR₃R₄ stehen,
R₂ C₄-C₁₂-Alkyl ist,
R₃ und R₄ unabhängig voneinander C₁-C₁₂-Alkyl bedeuten und
R₅ C₁-C₁₈-Alkyl ist.
R₅ C₁-C₁₈-Alkyl ist.

4. Verbindungen nach Anspruch 1, worin n = 2 ist, R₂ für Octyl oder CH₂-CO-O-R₅ steht, und R₁, R₃, R₄, und R₅ n-Octyl, 2-Ethylhexyl oder Isooctyl sind.

5. Verbindungen nach Anspruch 1, worin n = 1 ist, R und R^{●} für -SR₂ steht und R₂ CH₂-CO-O-iso-Octyl ist.

6. Zusammensetzung enthaltend
A) einen Schmierstoff, eine Hydraulik- oder Metallbearbeitungsflüssigkeit und
B) mindestens eine Verbindung der Formel I, worin R₁ zusätzlich Wasserstoff bedeutet.

7. Zusammensetzung nach Anspruch 6, die zusätzlich ein oder mehrere weitere Additive aus der Reihe der Korrosionsschutzmittel, Rostinhibitoren, Metall-Desaktivatoren, Viskositätsindex-Verbesserer, Dispergiermittel, Antioxidantien, Stockpunkterniedriger, Hochdruck- und Verschleißschutzzusätze enthält.

8. Verwendung von Verbindungen der Formel I gemäß Anspruch 1 als Additive für Schmierstoffe, Hydraulik- oder Metallbearbeitungsflüssigkeiten.

9. Verwendung einer Schmierstoffzusammensetzung nach Anspruch 7 als Motorenöl.

10. Verfahren zur Verbesserung der Gebrauchseigenschaften von Schmierstoffen, Hydraulik- oder Metallbearbeitungsflüssigkeiten, dadurch gekennzeichnet, daß diesen mindestens eine Verbindung der Formel I, wie in Anspruch 1 beschrieben, zugefügt wird.

## Claims

1. A compound of formula I 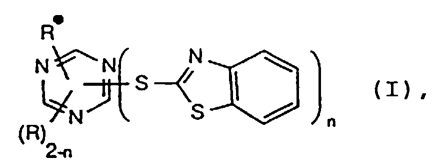 wherein
n is 1 or 2,
R and R^{●} are each independently of the other -OR₁, -SR₂ or -NR₃R₄
R₁ is C₁-C₃₀alkyl, C₅-C₇cycloalkyl, phenyl or naphthyl, phenyl- or naphthyl-substituted C₁-C₁₈alkyl or phenyl or naphthyl which are substituted by C₁-C₁₂alkyl, C₁-C₁₂alkoxy or C₁-C₁₂hydroxyalkyl,
R₂ is C₁-C₃₀alkyl, phenyl or naphthyl, phenyl- or naphthyl-substituted C₁-C₁₈alkyl or phenyl or naphthyl which are substituted by C₁-C₁₂alkyl, C₁-C₁₂alkoxy or C₁-C₁₂hydroxyalkyl, or is a radical of formula (CH₂)ₚ-CO-OR₅,
p is 1 or 2,
R₃ and R₄ have each independently of the other the meanings of R₁, or -NR₃R₄ is piperidyl, pyrrolidyl or azepyl, and
R₅ is hydrogen, C₁-C₃₀alkyl, C₅-C₇cycloalkyl, phenyl or naphthyl, phenyl- or naphthyl-substituted C₁-C₁₈alkyl or phenyl or naphthyl which are substituted by C₁-C₁₂alkyl, C₁-C₁₂alkoxy or C₁-C₁₂hydroxyalkyl.

2. A compound according to claim 1, wherein R₁ is C₁-C₁₈alkyl, R₂ is C₁-C₁₈alkyl or CH₂-CO-OR₅, R₃ and R₄ are each independently of the other C₁-C₁₈alkyl or benzyl, or -N₃R₄ is piperidyl, pyrrolidyl or azepyl, and R₅ is C₁-C₁₈alkyl.

3. A compound according to claim 2, wherein R and R^{●} are each independently of the other -SR₂ or -NR₃R₄, R₂ is C₄-C₁₂alkyl, R₃ and R₄ are each independently of the other C₁-C₁₂alkyl, and R₅ is C₁-C₁₈alkyl.

4. A compound according to claim 1, wherein n = 2, R₂ is octyl or CH₂-CO-O-R₅, and R₁, R₃, R₄ and R₅ are n-octyl, 2-ethylhexyl or isooctyl.

5. A compound according to claim 1, wherein n - 1, R and R^{●} are -SR₂ and R₂ is CH₂-CO-O-isooctyl.

6. A composition comprising
A) a lubricant, a hydraulic fluid or a metalworking fluid, and
B) at least one compound of formula I in which R₁ is additionally hydrogen.

7. A composition according to claim 6, which additionally comprises one or more further additives selected from the group consisting of corrosion inhibitors, rust inhibitors, metal deactivators, viscosity index improvers, dispersants, antioxidants, pour-point depressants, extreme-pressure and antiwear additives.

8. The use of a compound of formula I according to claim 1 as additive for lubricants, hydraulic fluids or metalworking fluids.

9. The use of a lubricant composition according to claim 7 as engine oil.

10. A process for enhancing the performance properties of lubricants, hydraulic fluids or metalworking fluids, which comprises adding thereto at least one compound of formula I as described in claim 1.

## Revendications

1. Composés de formule I 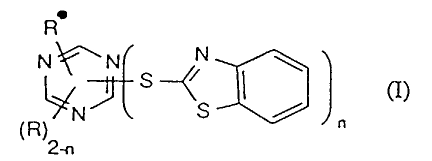 dans laquelle
n vaut 1 ou 2,
R et R^{●} représentent chacun indépendamment de l'autre -OR₁, -SR₂ ou -NR₃R₄,
R₁ est un radical alkyle en C₁-C₃₀, cycloalkyle en C₅-C₇, phényle ou naphtyle, alkyle en C₁-C₁₈ substitué par des substituants phényle ou naphtyle, ou phényle ou naphtyle substitué par des substituants alkyle, alcoxy ou hydroxyalkyle en C₁-C₁₂,
R₂ est un radical alkyle en C₁-C₃₀, phényle ou naphtyle, alkyle en C₁-C₁₈ substitué par des substituants phényle ou naphtyle, ou phényle ou naphtyle substitué par des substituants alkyle, alcoxy ou hydroxyalkyle en C₁-C₁₂, ou encore représente un radical de formule (CH₂)ₚ-CO-OR₅,
p vaut 1 ou 2,
R₃ et R₄, indépendamment l'un de l'autre, ont les significations de R₁, ou encore
-NR₃R₄ représentent le groupe pipéridyle, pyrrolidyle ou azépyle, et
R₅ est un hydrogène ou un radical alkyle en C₁-C₃₀, cycloalkyle en C₅-C₇, phényle ou naphtyle, alkyle en C₁-C₁₈ substitué par des substituants phényle ou naphtyle, ou encore phényle ou naphtyle substitué par des substituants alkyle, alcoxy ou hydroxyalkyle en C₁-C₁₂.

2. Composés de formule I, dans laquelle
R₁ est un radical alkyle en C₁-C₁₈,
R₂ est un radical alkyle en C₁-C₁₈ ou CH₂-CO-OR₅,
R₃ et R₄, indépendamment l'un de l'autre, sont des groupes alkyle en C₁-C₁₈ ou le radical benzyle, ou encore -NR₃R₄ représente le groupe pipéridyle, pyrrolidyle ou azépyle,
et R₅ est un radical alkyle en C₁-C₁₈.

3. Composés selon la revendication 2, dans lesquels R et R^{●} , chacun indépendamment de l'autre, représentent -SR₂ ou -NR₃R₄,
R₂ est un radical alkyle en C₄-C₁₂,
R₃ et R₄, indépendamment l'un de l'autre, sont chacun un radical alkyle en C₁-C₁₂, et
R₅ est un radical alkyle en C₁-C₁₈.

4. Composés selon la revendication 1, dans lesquels n vaut 2, R₂ est le radical octyle ou un radical CH₂-CO-O-R₅, et R₁, R₂, R₃ et R₄ sont les radicaux n-octyle, 2-éthylhexyle ou isooctyle.

5. Composés selon la revendication 1, dans lesquels n vaut 1, R et R^{●} sont -SR₂, et R₂ est le groupe CH₂-CO-O-iso-octyle.

6. Composition contenant
A) un lubrifiant, un fluide hydraulique ou un liquides pour l'usinage des métaux, et
B) au moins un composé de formule I dans laquelle R₁ représente en outre l'hydrogène.

7. Composition selon la revendication 6, qui contient en outre un ou plusieurs autres additifs choisis parmi l'ensemble comprenant les inhibiteurs de corrosion, les anti-rouilles, les désactivateurs de métaux, les agents améliorant l'indice de viscosité, les dispersants, les anti-oxydants, les agents abaissant le point d'écoulement, les additifs haute pression et les additifs anti-usure.

8. Utilisation de composés de formule I selon la revendication 1 comme additifs pour lubrifiants, fluides hydrauliques ou liquides pour l'usinage des métaux.

9. Utilisation d'une composition lubrifiante selon la revendication 7 en tant qu'huile moteur.

10. Procédé pour améliorer les propriétés à l'usage de lubrifiants, fluides hydrauliques ou liquides pour l'usinage des métaux, caractérisé en ce qu'on leur ajoute au moins un composé de formule I tel que décrit dans la revendication 1.
